# EUROPÄISCHE PATENTSCHRIFT

(11) **EP 2 012 677 B1**
(45) Veröffentlichungstag und Bekanntmachung des Hinweises auf die Patenterteilung: **16.11.2011**
(21) Anmeldenummer: 07701322.5
(22) Anmeldetag: 14.02.2007
(51) Int. Cl.: A61B 17/068

(54) **VORRICHTUNG ZUM SETZEN VON BEFESTIGUNGSANKERN IN DAS MENSCHLICHE ODER TIERISCHE GEWEBE**
DEVICE FOR INSERTING SECURING ANCHORS INTO HUMAN OR ANIMAL TISSUE
DISPOSITIF DE POSE D'ÉLÉMENTS D'ANCRAGE DANS DES TISSUS HUMAINS OU ANIMAUX

(30) Priorität: 28.02.2006 AT 3332006
(43) Veröffentlichungstag der Anmeldung: 14.01.2009
(73) Patentinhaber: Bard Shannon Limited, 3231 HR Nieuwegein (NL)
(72) Erfinder: EGLE, Walter, A-6842 Koblach (AT); ERHARD, Martin, A-6780 Silbertal (AT)
(74) Vertreter: Hofmann, Ralf U.
(86) Internationale Anmeldenummer: PCT/AT2007/000078
(87) Internationale Veröffentlichungsnummer: WO 2007/098512

(56) Entgegenhaltungen:
- DE-A1- 10 300 787
- US-A1- 2005 240 222

## Beschreibung

Die Erfindung betrifft eine zum Setzen von Befestigungsankern in das menschliche oder tierische Gewebe, insbesondere zur Befestigung von Kunststoffnetzen, mit einem Betätigungsteil, das für einen Setzvorgang von einer Ausgangsstellung zu einer Vorschubstellung in eine Vorschubrichtung vorschiebbar und entgegen der Vorschubrichtung zu seiner Ausgangsstellung rückstellbar ist, einer vom Betätigungsteil bei dessen Vorschub von der Ausgangsstellung zur Vorschubstellung vorgeschobenen und bei dessen Rückstellung von der Vorschubstellung zur Ausgangsstellung zurückgezogenen Nadel, die am bezogen auf die Vorschubrichtung vorderen Ende eine Nadelspitze besitzt, mehreren Befestigungsankern, die jeweils einen in das Gewebe einzudrückenden Schaft, mindestens einen gegenüber dem Schaft vorspringenden Teil mit einer dem vorderen Ende der Nadel abgewandten Rückseite und einen den Befestigungsanker durchsetzenden Durchgangskanal aufweisen und die auf der ihre Durchgangskanäle durchsetzenden Nadel angeordnet sind, einem beim Vorschub des Betätigungsteils von dessen Ausgangsstellung zur Vorschubstellung vorgeschobenen Eindrückteil zum Eindrücken des jeweils bezogen auf die Vorschubrichtung vordersten, in einer Bereitschaftsposition im Bereich der Nadelspitze sich befindenden Befestigungsankers ins Gewebe und einem Nachführmechanismus zum in Richtung zur Nadelspitze gerichteten Verschieben der hinter dem ins Gewebe einzudrückenden Befestigungsanker sich befindenden Befestigungsanker beim Setzvorgang.

Kunststoffnetze werden beispielsweise bei Bruchoperationen eingesetzt und müssen hierbei am menschlichen Gewebe befestigt werden. Außer Befestigungen durch Vernähen sind verschiedene Befestigungselemente bekannt geworden, beispielsweise Befestigungsanker, die einen in das menschliche Gewebe einzudrückenden Schaft und einen über den Schaft vorspringenden tellerförmigen Teil zur Halterung des Kunststoffnetzes aufweisen. Zum Setzen bzw. Eintreiben von solchen Befestigungsankern sind verschiedene Vorrichtungen bekannt geworden.

Eine Vorrichtung der eingangs genannten Art ist aus der DE 103 00 787 A1 bekannt. Das Eindrückteil besitzt hier eine auf der Nadel hinter dem letzten Befestigungsanker angeordnete Lochscheibe. An gegenüberliegenden Rändern dieser Lochscheibe sind entgegen der Vorschubrichtung sich erstreckende erste und zweite Federlappen angebracht, deren Enden mit Rastrippen eines Halters für die Nadel zusammenwirken. Weiters sind an der Lochscheibe gegenüberliegende dritte und vierte Federlappen angebracht, die kürzer als die ersten und zweiten Federlappen ausgebildet sind, sich ebenfalls entgegen der Vorschubrichtung erstrecken und mit Rastöffnungen in einer Außenhülse der Vorrichtung zusammenwirken. Das Betätigungsteil ist mit dem Halter für die Nadel gekoppelt und bei einem Vorschub des Betätigungsteils wird der Nadelhalter und mit ihm das Eindrückteil, dessen längere Federlappen an den Rastrippen des Halters angreifen, vorgeschoben. Das Eindrückteil schiebt somit die auf der Nadel verschiebbar angeordneten Befestigungsanker ebenfalls vor, wobei der vorderste Befestigungsanker in das Gewebe eingedrückt wird. Bei der Rückstellung des Betätigungsteils und des Halters für die Nadel kommt es zum Rasteingriff zwischen den kürzeren Federlappen und einer nächstvorderen Rastöffnung in der Außenhülse, wodurch eine weitere Rückstellung des Eindrückteils blockiert wird und das Eindrückteil gegenüber der Nadel in Richtung deren vorderer Spitze verschoben wird. Hierbei gelangen die längeren Federlappen des Eindrückteils mit der nächstvorderen Rastrippe des Halters der Nadel in Eingriff. Dieser Vorgang wiederholt sich bei jedem Setzvorgang, wobei das Eindrückteil und die verbliebenen Befestigungsanker immer weiter in Richtung der Spitze der Nadel rücken, bis der letzte Befestigungsanker gesetzt worden ist. Nachteilig bei dieser Vorrichtung ist es, dass die Höchstzahl der Befestigungsanker, die anfänglich auf der Nadel angeordnet werden können, relativ gering ist, beispielsweise 10 bis 20 Stück beträgt, da bei einer höheren Anzahl von Befestigungsankern aufgrund der Elastizität der Teile ein ordnungsgemäßes Funktionieren des Nachführmechanismus für die Befestigungsanker nicht mehr gegeben ist. Auch ist die gesamte Baulänge der Vorrichtung relativ groß, da für den Nachführmechanismus zumindest noch einmal die gleiche Baulänge vorgesehen werden muss, welche die anfangs auf der Nadel angeordneten Befestigungsanker einnehmen.

Die DE 199 35 904 C1 beschreibt eine Einrichtung zum Setzen von Clips, welche zusammendrückbare Spitzen oder Krallen aufweisen, beispielsweise um ein zur Stabilisierung dienendes Netz mit dem Gewebe zu verbinden. Die Clips sind in einem Magazin enthalten. Zum Setzen eines Clips dient ein Schubdraht, wobei ein Stempel am vordersten Clip angreift und diesen verschwenkt und zum distalen Ende der Applikatorspitze führt. Ab dieser Position ist der Stempel durch eine Federverbindung mit dem Schubdraht von einer weiteren Vorwärtsbewegung des Schubdrahts abgekoppelt. Der Clip wir nunmehr an der vorgesehenen Stelle im Gewebe positioniert. Beim weiteren Ziehen des Handgriffs werden die Spitzen oder Krallen des Clips durch Zusammenwirken des Stempels mit einem Kniegelenk im durchdrungenen Gewebe geschlossen und eine Rückhalteklammer für den Clip wird freigegeben, so dass die Applikatorspitze zurückgezogen werden kann. Die dem gesetzten Clip nachfolgenden Clips werden durch Federkraft zum distalen Ende des Magazins nachgeführt. Es handelt sich um eine nicht gattungsgemäße Einrichtung ohne eine zentrale Führung für Befestigungsanker durch eine Nadel, um ein achsiales Eindrücken eines Befestigungsankers sicherzustellen.

Eine weitere nicht gattungsgemäße Einrichtung ohne eine zentrale Führung für einen Befestigungsanker ist aus der US 6,277,131 B1 bekannt. Zum Setzen von aus einem Magazin zugeführten Clips nimmt ein distaler Finger eines Vorschubteils den vordersten Clip mit, um ihn zwischen Backen des Instruments einzusetzen. Ein proximaler Finger wird hierbei zunächst in einer Ausnehmung einer Leiter verschoben, um dann einen Vorschub der Leiter und somit eine Nachführung der nachfolgenden Clips zu bewirken. Toleranzen der im Magazin angeordneten Clips sollen auf diese Weise ausgeglichen werden.

Aufgabe der Erfindung ist es, eine verbesserte Vorrichtung der eingangs genannten Art bereitzustellen, die eine relativ geringe Baulänge aufweist und auch für eine höhere Anzahl an Befestigungsankern ausgelegt werden kann. Erfindungsgemäß gelingt dies durch eine Vorrichtung mit den Merkmalen des Anspruchs 1.

Gemäß der Erfindung greift das Eindrückteil somit nicht am letzten auf der Nadel angeordneten Befestigungsanker, sondern an einem weiter vorne liegenden, vorzugsweise am vordersten, der auf der Nadel angeordneten Befestigungsanker an. Bei der Rückstellung des Betätigungsteils nach dem Setzen des vordersten Befestigungsankers werden die Anlageelemente des Eingriffsteils mit dem nächsthinteren auf der Nadel angeordneten Befestigungsanker in Eingriff gebracht und greifen somit beim nächsten Setzvorgang an diesem an. Die Funktion des Nachstellmechanismus kann hierbei von der Anzahl der hinter dem Befestigungsanker, an welchem das Eindrückteil angreift, liegenden Befestigungsanker unabhängig sein, so dass sich die Vorrichtung auch für eine größere Anzahl von auf der Nadel angeordneten Befestigungsankern eignet. Weiters wird durch die erfindungsgemäße Ausbildung, bei der das Eindrückteil bei jedem Setzvorgang mit dem nächsthinteren, auf der Nadel in Richtung zu deren Spitze nachrückenden Befestigungsanker in Eingriff gebracht wird, eine relativ kurze Baulänge der Vorrichtung ermöglicht.

Erfindungsgemäß kann weiters eine sehr direkte Kraftübertragung auf den zu setzenden Befestigungsanker erreicht werden, die das Setzen erleichtert.

In einer vorteilhaften Ausführungsform der Erfindung umfasst der Nachführmechanismus eine am hintersten auf der Nadel angeordneten Befestigungsanker angreifende Feder, welche diesen Befestigungsanker in die Vorschubrichtung beaufschlagt. Das Eindrückteil weist hierbei bevorzugterweise mindestens eine federelastische Haltezunge auf, welche in der Ausgangsstellung des Betätigungsteils mit mindestens einem vorspringenden Teil desjenigen Befestigungsankers zusammenwirkt, der dem Befestigungsanker nachfolgt, an dem die Angriffselemente des Eindrückteils angreifen. Die mindestens eine federelastische Haltezunge kann diesen nachfolgenden Befestigungsanker gegen die Federkraft der Feder des Nachführmechanismus zurückhalten.

Vorzugsweise ist das Eindrückteil hülsenförmig ausgebildet und die federelastischen Haltezungen erstrecken sich schräg nach innen und vorne (bezogen auf die Vorschubrichtung).

Eine vorteilhafte Ausführungsform der Erfindung sieht vor, dass das Eindrückteil verschiebbar in einer Außenhülse angeordnet ist, welche mindestens eine federelastische Haltezunge aufweist, die in der Ausgangsstellung des Betätigungsteils mit mindestens einem vorspringenden Teil des vordersten auf der Nadel angeordneten Befestigungsankers zusammenwirkt, um diesen gegen eine Verschiebung in die Vorschubrichtung zurückzuhalten.

Vorzugsweise besitzt die Außenhülse weiters mindestens ein Sperrelement, von dem bei der Rückstellung des Betätigungselements von seiner Vorschubstellung zu seiner Ausgangsstellung eine Verschiebung des nächsthinteren Befestigungsankers entgegen der Vorschubrichtung blockiert wird, wobei eine Überführung der Angriffselemente des Eindrückteils über oder um mindestens eine vorspringenden Teil des nächsthinteren Befestigungsankers erfolgt. Durch die Blockierung des Befestigungsankers bei der Rückstellung des Betätigungsteils erfolgt weiters eine Überführung der mindestens einen federelastischen Haltezunge des Eindrückteils über oder um den mindestens einen vorspringenden Teil des Befestigungsankers.

Wenn in dieser Schrift von "vorne" und "hinten" die Rede ist, so ist dies auf die Vorschubrichtung des Betätigungsteils bzw. auf die Richtung bezogen, in welche der Befestigungsanker gesetzt wird. Die Angaben "außen" und "innen" sind auf die zentrale Längsachse der Vorrichtung bezogen, d.h. ein weiter außen liegendes Teil besitzt einen größeren Abstand von der zentralen Längsachse als ein weiter innen liegendes Teil.

Weitere Vorteile und Einzelheiten der Erfindung werden im Folgenden anhand der beiliegenden Zeichnung erläutert. In dieser zeigen:
- Fig. 1: eine Schrägsicht eines Ausführungsbeispiels einer erfindungsgemäßen Vorrichtung;
- Fig. 2: die Vorrichtung im abgenommenen Zustand eines vorderen Endstücks;
- Fig. 3: das Endstück im auseinandergezogen dargestellten Zustand von Teilen desselben, in Schrägsicht;
- Fig. 4: einen Befestigungsanker in Schrägsicht;
- Fig. 5: eine Seitenansicht der auf der Nadel anzuordnenden Feder;
- Fig. 6: eine Schrägsicht der Nadel und des Nadelhalters;
- Fig. 7 bis Fig. 9: Längsschnitte durch das Endstück in der Ausgangsstellung des Betätigungsteils, wobei die Schnittebene jeweils um 120° gedreht ist;
- Fig. 10 bis Fig. 12: Längsschnitte entsprechend den Fig. 7 bis 9, aber in der Vorschubstellung des Betätigungsteils;
- Fig. 13 bis Fig. 15: vergrößerte Ausschnitte der Fig. 7 bis 9;
- Fig. 16 bis Fig. 18: vergrößerte Ausschnitte der Fig. 10 bis 12;
- Fig. 19: ein Krallteil eines Nachstellmechanismus gemäß einer weiteren Ausführungsform der Erfindung.

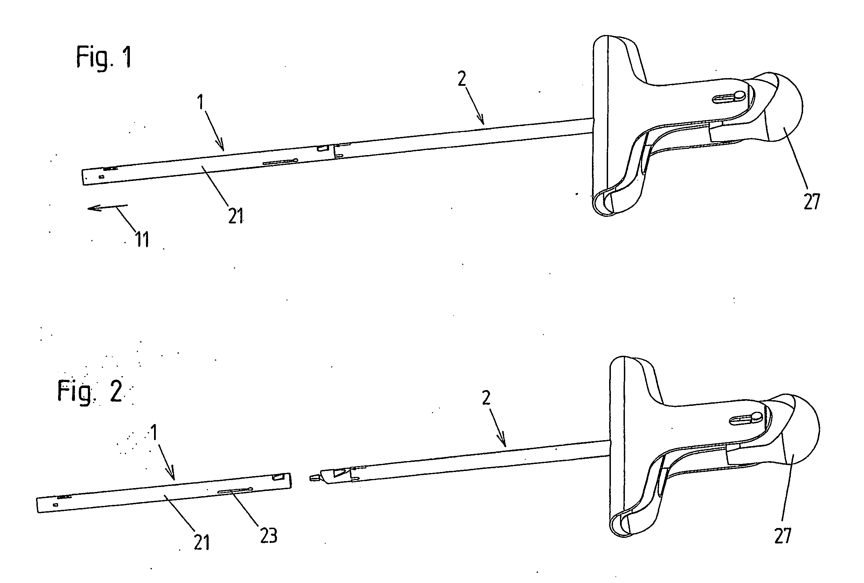

Ein Ausführungsbeispiel einer Vorrichtung bzw. eines medizinischen Instruments gemäß der Erfindung ist in den Fig. 1 bis 18 dargestellt. Die Fig. 1 und 2 zeigen Gesamtdarstellungen der Vorrichtung bzw. des medizinischen Instruments, welches auch als Applikator bezeichnet werden könnte. In Fig. 1 ist das medizinische Instrument hierbei zusammengesetzt dargestellt und in Fig. 2 ist ein Endstück 1, welches Befestigungsanker enthält, die zum Setzen bzw. Applizieren in menschliches oder tierisches Gewebe vorgesehen sind, von einem Bedienstück 2 abgekoppelt dargestellt.

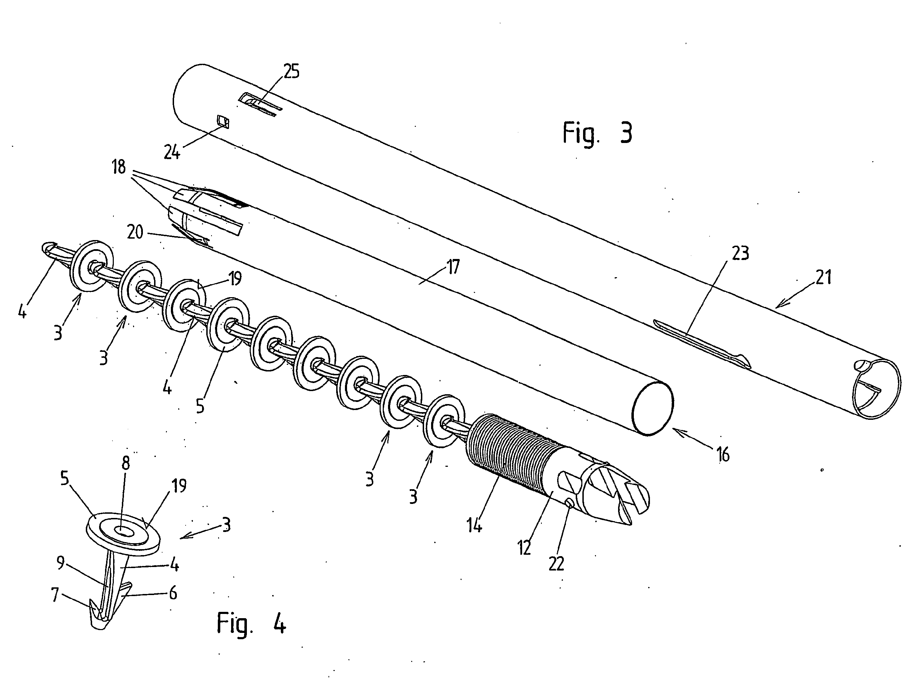

Der bei diesem Ausführungsbeispiel verwendete Befestigungsanker 3, auch Pin genannt, ist in Fig. 4 dargestellt und umfasst einen in das menschliche Gewebe einzudrückenden Schaft 4 und am Ende des Schafts 4 einen gegenüber des Schafts 4 nach außen vorspringenden, insbesondere tellerförmig ausgebildeten Teil, der zur Anlage an das vom Befestigungsanker 3 am Gewebe zu befestigende Teil dient, insbesondere eines bei einer Operation anzubringenden Kunststoffnetzes. Zur Verankerung im Gewebe besitzt der Schaft 4 widerhakenartige Vorsprünge 6, 7. Der Befestigungsanker 3 besitzt weiters einen diesen durchsetzenden Durchgangskanal 8, mit dem er auf eine Nadel aufgeschoben werden kann. Vorzugsweise ist im Bereich hinter einem widerhakenartigen Vorsprung eine Öffnung 9 im Mantel des Schaftes 4 ausgebildet, wodurch die Halterung des Befestigungsankers 3 im Gewebe verbessert wird (da Gewebe in die Öffnung 9 einfallen kann). Derartig ausgebildete Befestigungsanker 3 sind bekannt. Der Befestigungsanker 3 könnte auch eine demgegenüber modifizierte Ausbildung mit einem anders als tellerförmig ausgebildeten nach außen vorspringenden Teil oder mehrerer solcher nach außen vorspringender Teile aufweisen.

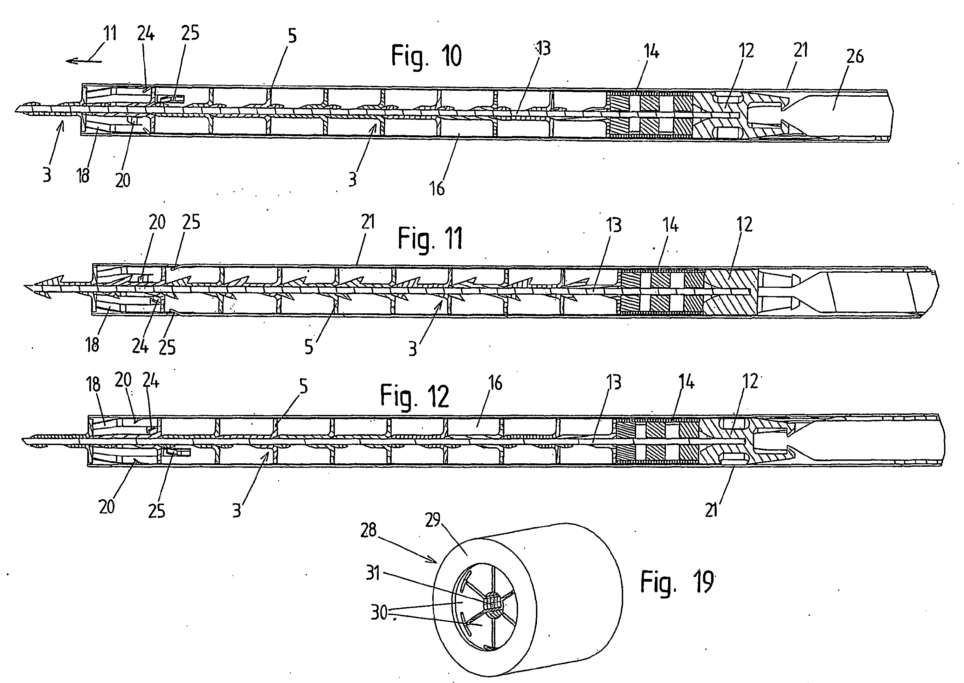

Die Vorrichtung umfasst ein Betätigungsteil 10, welches zwischen einer in den Fig. 2 und 7 bis 9 dargestellten Ausgangsstellung und einer in den Fig. 10 bis 12 dargestellten Vorschubstellung verschiebbar ist, und zwar in eine Vorschubrichtung 11 und entgegen derselben. Das Betätigungsteil 10 ist, wenn das Endstück 1 mit dem Bedienstück 2 gekoppelt ist, bezüglich der Vorschubrichtung unverschiebbar mit einem Nadelhalter 12 verbunden. Vorzugsweise ist diese Verbindung zwischen dem Betätigungsteil 10 und dem Nadelhalter 12 formschlüssig, beispielsweise in Form einer Rastverbindung. Am Nadelhalter 12 ist die Nadel 13 starr befestigt. Bei der Verschiebung in und entgegen der Vorschubrichtung 11 nimmt das Betätigungsteil 10 somit den Nadelhalter 12 und die Nadel 13 mit.

Die Nadel 13 besitzt an ihrem bezogen auf die Vorschubrichtung 11 vorderen Ende eine Spitze. Diese kann wie gezeigt in Form einer Stechspitze oder auch in Form einer Schneidspitze ausgebildet sein. Die zentrale Längsachse der Vorrichtung läuft durch die Nadel 13.

Auf der Nadel 13 sind eine Mehrzahl von Befestigungsankern 3 angeordnet, deren Durchgangskanäle 8 von der Nadel 13 durchsetzt werden, wobei die einzelnen auf der Nadel 13 hintereinander angeordneten Befestigungsanker 3 aneinander anliegen. Die Durchgangskanäle 8 der auf der Nadel 13 angeordneten Befestigungsanker 3 liegen somit parallel zur Nadel 13. Im gezeigten Ausführungsbeispiel ragt das vordere Ende des Schaftes 4 jeweils über ein kurzes Stück, welches kürzer als ¼ der Länge des gesamten Befestigungsankers ist, in einen vergrößerten Abschnitt des Durchgangskanals 8 am hinteren Ende des nächstvorderen Befestigungsankers 3.

Am hintersten Befestigungsanker 3 greift eine Feder 14 an, welche diesen und somit auch vor diesem liegende Befestigungsanker 3 in die Vorschubrichtung 11 beaufschlagt. Die im gezeigten Ausführungsbeispiel in Form einer Schraubenfeder 14 ausgebildete Feder stützt sich an ihrem hinteren Ende am Nadelhalter 12 ab.

Die Feder 14 ist mit mehreren Stützscheiben 15 versehen, die von der Nadel 13 durch eine Bohrung durchsetzt werden.

Am Nadelhalter 12 ist ein im gezeigten Ausführungsbeispiel hülsenförmig ausgebildetes Eindrückteil 16 in und entgegen der Vorschubrichtung 11 unverschiebbar festgelegt. Vorzugsweise ist das Eindrückteil 16 mit dem Nadelhalter 12 und somit auch mit der Nadel 13 starr verbunden, beispielsweise durch Verklebung mit dem Nadelhalter 12.

Das hülsenförmige Eindrückteil 16 weist einen Abschnitt 17 auf, der sich im vollständig mit Befestigungsankern 3 gefüllten Zustand der Vorrichtung zumindest über einige der Befestigungsanker in Richtung zum vorderen Ende der Nadel 13 erstreckt und an dessen vorderem Ende Angriffselemente 18 zum Angriff an der Rückseite 19 des vorspringenden Teils 5 eines Befestigungsankers 3 angeordnet sind. Im gezeigten Ausführungsbeispiel ist der Befestigungsanker 3, an dem die Angriffselemente 18 beim Vorschub des Betätigungsteils 10 angreifen, der vorderste auf der Nadel 13 angeordnete Befestigungsanker 3, was bevorzugt ist.

Die Angriffselemente 18 sind zungenförmig und werden bevorzugterweise gebildet, indem vom vorderen Ende des hülsenförmigen Eindrückteils 16 ausgehende, in achsialer Richtung verlaufende Einschnitte eingebracht werden. Diese Zungen erstrecken sich bezogen auf die Vorschubrichtung 11 nach vorne, wobei sie einen schräg nach innen (also in Richtung zur Nadel 13) verlaufenden Abschnitt aufweisen, um mit ihrem vorderen Ende am tellerförmigen, vorspringenden Teil 5 des Befestigungsankers 3 anzuliegen. Die zungenförmigen Angriffselemente 18 sind federelastisch nach außen, also in Richtung von der Nadel 13 weg auslenkbar. Anstelle eines schräg nach innen verlaufenden Abschnitts könnten diese zungenförmigen Angriffselemente 18 auch insgesamt schräg nach innen verlaufend ausgebildet sein.

Im Bereich des vorderen Endes des Eindrückteils 16 besitzt dieses weiters zwei gegenüberliegende federelastische Haltezungen 20. Diese wirken mit dem vorspringenden Teil 5 desjenigen Befestigungsankers 3 zusammen, der dem Befestigungsanker 3 nachfolgt, an dem die Angriffselemente 18 des Eindrückteils 16 angreifen. Im gezeigten Ausführungsbeispiel wirken die Haltezungen 20 also mit dem zweitvordersten auf der Nadel 13 angeordneten Befestigungsanker 3 zusammen. Die Haltezungen 20 weisen zumindest einen Abschnitt auf, der schräg nach vorne und innen verläuft oder verlaufen insgesamt schräg nach vorne und innen.

Das Eindrückteil 16 ist verschiebbar in einer Außenhülse 21 angeordnet. Zur verschiebbaren Lagerung der aus dem Eindrückteil 16, der Nadel 13 und dem Nadelhalter 12 bestehenden Einheit in der Außenhülse 21 sind im gezeigten Ausführungsbeispiel am Nadelhalter 12 gegenüberliegende Führungszapfen 22 vorgesehen, die in in Längsrichtung der Außenhülse 21 verlaufende Führungsschlitze 23 eingreifen.

In der Ausgangsstellung des Betätigungsteils 10 umgibt die Außenhülse 21 alle auf der Nadel 13 angeordnete Befestigungsanker 3.

In ihrem vorderen Endbereich besitzt die Außenhülse 21 gegenüberliegende federelastische Haltezungen 24, die in der Ausgangsstellung des Betätigungsteils 10 mit dem vorspringenden Teil 5 des vordersten auf der Nadel 13 angeordneten Befestigungsankers 3 zusammenwirken und dessen Verschiebung in die Vorschubrichtung 11 verhindern. Die Haltezungen 24 weisen zumindest einen Abschnitt auf, der sich schräg nach vorne und innen erstreckt oder strecken sich insgesamt schräg nach vorne und innen.

Weiters weist die Außenhülse 21 gegenüberliegende Sperrelemente 25 auf. Deren vordere Enden befinden sich weiter hinten als die Haltezungen 24. Im gezeigten Ausführungsbeispiel sind die Sperrelemente 25 in Form von Federzungen ausgebildet, die sich nach vorne erstrecken und einen schräg nach innen, d.h. der Nadel 13 sich annähernden Endabschnitt aufweisen. Sie könnten auch insgesamt (über ihre gesamte Länge) schräg nach vorne und innen verlaufend ausgebildet sein.

Bei der gezeigten Ausführungsform, bei der das vordere Endstück 1 an- und abkoppelbar mit dem Bedienstück 2 verbunden ist, um eine Auswechslung des vorderen Endstücks 1 nach dem Aufbrauch der Befestigungsanker 3 zu ermöglichen, ist das Betätigungsteil 10 beispielsweise über einen Rastmechanismus mit dem Nadelhalter 12 verbunden. Damit sich beim Aufstecken des Endstücks 1 auf das Bedienstück 2, bei welchem die Verrastung des Nadelhalters 12 mit dem Betätigungsteil 10 erfolgt, die Nadel 13 und das Eindrückteil 16 nicht nach vorne verschieben, weisen die Führungsschlitze 23 Verengungen auf. Die Kraft, um die Führungszapfen 22 durch diese Verengungen zu führen, ist größer als die zum Verrasten des Betätigungsteils 10 mit dem Nadelhalter 12 erforderliche Kraft.

Das am vorderen Ende eines Übertragungsschafts 26 des Bedienstücks 2 angeordnete Betätigungsteil 10 kann mittels einer Handhabe 27 des Bedienstücks 2, die gegen die Kraft einer in den Figuren nicht dargestellten Feder in die Vorschubrichtung 11 verstellbar ist und von der Feder entgegen der Vorschubrichtung rückstellbar ist, zwischen seiner Ausgangsstellung und seiner Vorschubstellung verschoben werden.

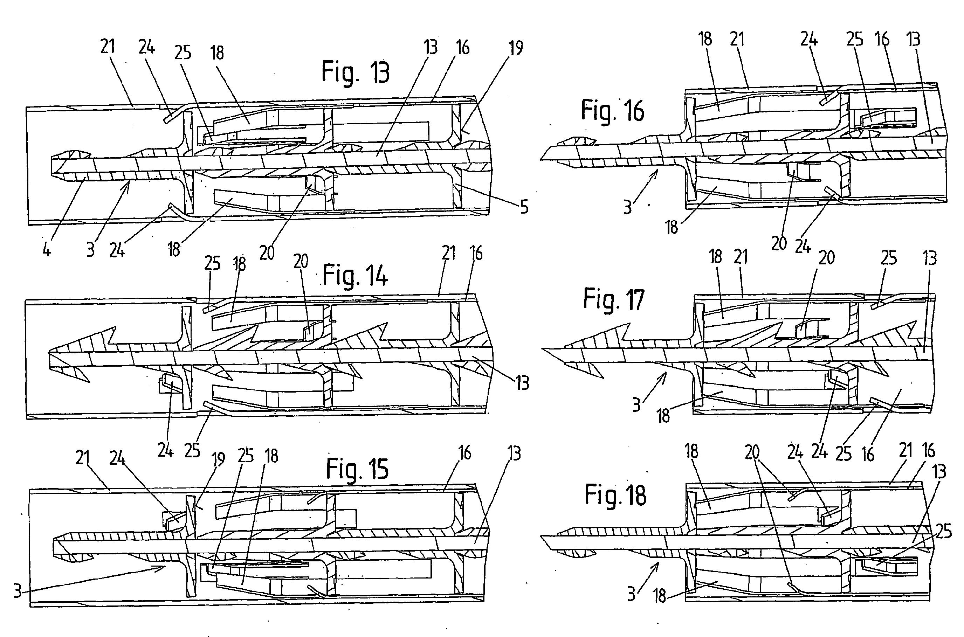

Wird zum Setzen eines Befestigungsankers 3 das Betätigungsteil 10 ausgehend von seiner Ausgangsstellung in die Vorschubrichtung verschoben, so werden mit diesem die Nadel 13 und das Eindrückteil 16 ebenfalls in die Vorschubrichtung 11 verschoben. Mittels der Eingriffselemente 18, die vorzugsweise direkt am vordersten Befestigungsanker 3 angreifen, wird der sich in seiner Bereitschaftsposition im Bereich der Nadelspitze befindende vorderste Befestigungsanker 3 an den Haltezungen 21 vorbeigeführt, wobei diese nach außen ausgelenkt werden, und zusammen mit dem vorderen Endabschnitt der Nadel 13 aus dem vorderen Ende der Außenhülse 21 hinausgeschoben. Die Spitze der Nadel 13 läuft hierbei dem vorderen Ende des Schafts 4 bevorzugterweise etwas vor (vgl. Fig. 16 bis 18). Da die Außenhülse 21 beim Setzvorgang auf das Gewebe, in welches der Befestigungsanker 3 einzudrücken ist, aufgesetzt wird, wird hierbei das Gewebe von der Spitze der Nadel 13 vorgestochen bzw. vorgeschnitten und der Schaft 4 des Befestigungsankers 3 in das Gewebe eingedrückt.

Die Haltezungen 20 sind zur Sicherheit vorgesehen, damit der vorderste Befestigungsanker 3 nicht gleich vollständig aus der Außenhülse 21 herausgedrückt wird, wenn diese während des Setzvorgangs nicht immer auf das Gewebe aufgesetzt ist. Die Haltezungen 20 sind hierfür so starr, dass sie einen Befestigungsanker 3 gegen die Kraft der Feder 14 halten können. Durch die Kraft der Feder 14 rücken die hinter dem von den Angriffselementen 18 beaufschlagten Befestigungsanker 3 liegenden Befestigungsanker 3 in die Vorschubrichtung nach, wobei der nächsthintere Befestigungsanker 3 unter Auslenkung der Sperrelemente 25 an diesen vorbeigeführt wird.

In der Vorschubstellung des Betätigungsteils 10 ist die vollständig vorgeschobene Stellung der Nadel 13 und des vordersten Befestigungsankers 3 erreicht, der bis zu seinem vorspringenden Teil 5, welches sich am vorderen Ende der Außenhülse 21 befindet, in das Gewebe eingedrückt worden ist. In dieser vollständig vorgeschobenen Stellung kann die Haltezunge 20 vom vorspringenden Teil 5 des hinter dem beaufschlagten Befestigungsanker 3 liegenden Befestigungsankers 3 abgehoben sein, wie dies aus den Fig. 10 bis 12 bzw. 16 bis 18 ersichtlich ist. Die Haltezungen 24 halten diesen Befestigungsanker 3 dann gegen die Kraft der Feder 14 zurück und sind hierzu entsprechend stark ausgebildet.

Nachdem die vollständig vorgeschobene Stellung der Nadel 13 und des vordersten Befestigungsankers 3 erreicht ist, wobei sich das Betätigungsteil 10 in seiner Vorschubstellung befindet (vgl. Fig. 10 bis 12 und 16 bis 18), wird das Betätigungsteil 10 entgegen der Vorschubrichtung 11 zurückgezogen, beispielsweise durch die auf die Handhabe 27 wirkende Feder. Hierbei wird die Nadel 13 aus dem Durchgangskanal 8 des in das Gewebe eingedrückten Befestigungsankers 3 zurückgezogen. Für den dem eingedrückten Befestigungsanker 3 nachfolgenden Befestigungsanker 3 wird eine Verschiebung entgegen der Vorschubrichtung 11 blockiert, wenn es zur Anlage an den Sperrelementen 25 gelangt. Dadurch werden die bevorzugterweise vorgesehenen Haltezungen 20 beim Zurückziehen des Eindrückteils 16 nach außen ausgelenkt und über den vorspringenden Teil 5 des Befestigungsankers 3 überführt. Wenn der von den Sperrelementen 25 zurückgehaltene Befestigungsanker 3 beim Zurückziehen des Eindrückteils 16 über die Haltezunge 20 überführt worden ist, so wirkt auf diesen Befestigungsanker 3 wiederum die Kraft der Feder 14 und der Befestigungsanker 3 wird von der Feder gegen die Haltezunge 24 der Außenhülse 21 gedrückt, welche so stark ausgebildet sind, dass sie den Befestigungsanker 3 gegen die Kraft der Feder 14 zurückhalten können.

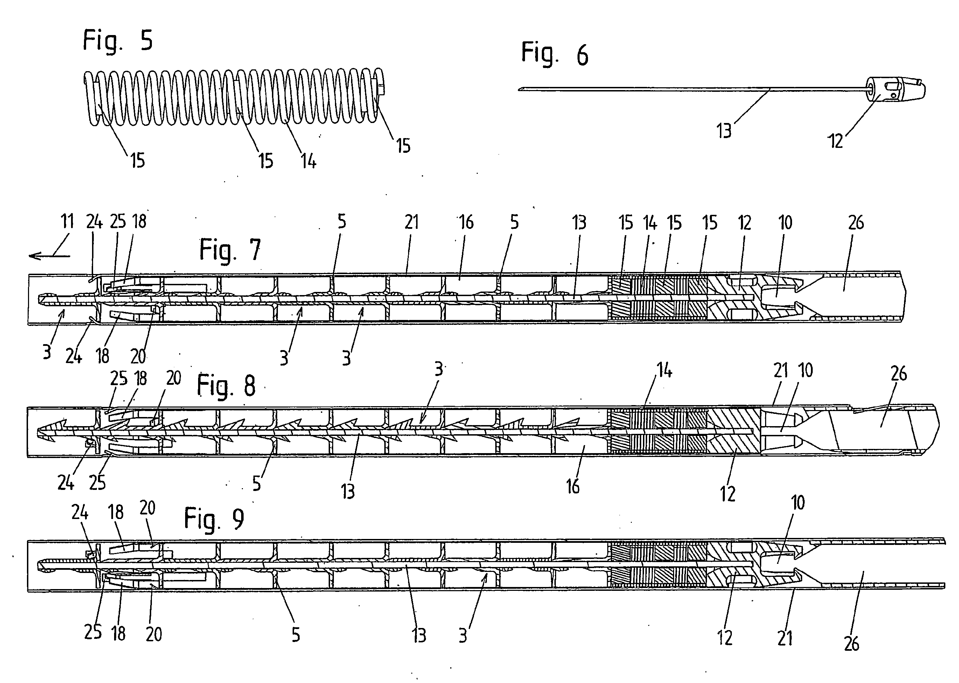

Weiters werden beim Zurückziehen des Eindrückteils 16 die zungenförmigen Angriffselemente 18 nach außen ausgelenkt und über den vorspringenden Teil 5 des Befestigungsankers 3 überführt. Wenn das Betätigungsteil 10 wiederum seine Ausgangsstellung erreicht hat, so ist der nunmehr vorderste Befestigungsanker 3 auf der Nadel 13 in Richtung zur Nadelspitze bis zur Bereitschaftsposition für den nächsten Setzvorgang verschoben worden. Die anderen Befestigungsanker 3 sind diesem durch die Kraft der Feder 14 nachgeschoben worden (vgl. Fig. 7 bis 9 und 13 bis 15).

Die Feder 14 hat sich gegenüber dem in den Fig. 7 bis 9 dargestellten Zustand nach dem Setzvorgang um die Länge eines Befestigungsankers 3 expandiert und eine weitere solche Expansion erfolgt bei jedem Setzen eines Befestigungsankers.

Bei zunehmender Expansion der Feder 14 würde ohne die Stützscheiben 15 die freie Knicklänge der Nadel 13 einen großen Wert erlangen. Durch die Stützscheiben 15, insbesondere die mindestens eine im Bereich der Mitte der Nadel angeordnete Stützscheibe 15 wird die Nadel 13 bei diesen Stützscheiben abgestützt, welche ihrerseits von der Außenhülse 21 abgestützt sind. Die freie Knicklänge der Nadel 13 wird dadurch verringert.

Der Nachstellmechanismus könnte auch ohne eine Feder 14 ausgebildet sein. Zu diesem Zweck könnte beispielsweise ein Krallteil 28, wie es in der Fig. 19 dargestellt ist, auf der Nadel 13 angeordnet werden. Das Krallteil 28 besitzt in mindestens einer Ebene mehrere von einem Außenteil nach innen abstehende Federlappen 30. Die Öffnung 31 zwischen diesen Federlappen 30 ist etwas geringer als der Durchmesser der Nadel 13. Vorzugsweise sind mehrere hintereinander angeordnete Ebenen bzw. Schichten von solchen Federlappen 30 vorhanden, wie dies in Fig. 19 dargestellt ist. Wenn das Krallteil 28 von der der Nadelspitze gegenüberliegenden Seite achsial auf die Nadel 13 aufgeschoben wird, so sind die an der Nadel 13 anliegenden Federlappen 30 zur Rückseite der Nadel 13 hin gebogen. Hierdurch kann das Krallteil 28 in Richtung zur Spitze der Nadel 13 entlang dieser mit geringer Kraft verschoben werden, eine Verschiebung in entgegengesetzter Richtung ist aber blockiert.

Dieses Krallteil 28 wird hinter dem hintersten Befestigungsanker 3 auf der Nadel 13 angeordnet. Weiters werden in der Außenhülse 21 eine Reihe von Sperrelementen für das Krallteil 28 vorgesehen, beispielsweise in Form von schräg in der Vorschubrichtung nach innen abstehenden Haltezungen. Die Haltezungen 20 und Sperrelemente 25 können in diesem Ausführungsbeispiel entfallen, während die Haltezungen 24 vorzugsweise nach wie vor vorhanden sind (aber schwächer ausgebildet sein können).

Bei einem Vorschub des Betätigungsteils 10 und mit ihm der Nadel 13 und des Eindrückteils 16 wird das Krallteil 28 ebenfalls in die Vorschubrichtung 11 vorgeschoben, da eine Verschiebung des Krallteils 28 gegenüber der Nadel 13 in Richtung zum hinteren Ende der Nadel 13 blockiert ist. Das Krallteil 28 verschiebt somit die hinter demjenigen Befestigungsanker 3, mit dem die Angriffselemente 18 des Eindrückteils 16 zusammenwirken (vorzugsweise dem vordersten Befestigungsanker 3), liegenden Befestigungsanker 3 ebenfalls in die Vorschubrichtung 11.

Bei einer Rückstellung des Betätigungsteils 10 und mit ihm der Nadel 13 und des Eindrückteils 16 entgegen der Vorschubrichtung 11 kommt das Krallteil 28 an einem Sperrelement der Außenhülse 21 zur Anlage, so dass dessen Verschiebung entgegen der Vorschubrichtung 11 blockiert ist. Es verschiebt sich dadurch zusammen mit den auf der Nadel 13 verbliebenen Befestigungsankern 3 in Richtung zur Spitze der Nadel. Hierbei werden bei der Rückstellung des Eindrückteils 16 die Angriffselemente 18 des Eindrückteils 16 über den vorspringenden Teil 5 des nächsthinteren Befestigungsankers 3 überführt.

Unterschiedliche Modifikationen der gezeigten Ausführungsbeispiele sind denkbar und möglich, ohne den Bereich der Erfindung zu verlassen. So wäre es prinzipiell beispielsweise denkbar und möglich, anstelle eines umfänglich vorspringenden Teils 5 einen jeweiligen Befestigungsanker 3 mit zwei oder mehr einzelnen, um den Umfang verteilten vorspringenden Teilen zu versehen. Die Angriffselemente 18 des Eindrückteils 16 könnten in diesem Fall bei der Rückstellung des Betätigungsteils 10 auch in Umfangsrichtung auslenkbar ausgebildet sein und somit um die vorspringenden Teile 5 des betreffenden Befestigungsankers 3 überführt werden.

Zwar ist es bevorzugt, dass die Angriffselemente 18 des Eindrückteils 16 mit dem vordersten auf der Nadel 13 angeordneten Befestigungsanker 3 zusammenwirken. Denkbar und möglich wäre es aber auch, dass die Angriffselemente 18 mit dem zweitvordersten oder einem noch weiter hinten liegenden Befestigungsanker 3 zusammenwirken. Der Abschnitt 17 des Eindrückteils 16 erstreckt sich aber, im mit Befestigungsankern 3 vollständig befüllten Zustand der Vorrichtung, zumindest über einige der Befestigungsanker 3 in Richtung zum vorderen Ende der Nadel.

Anstelle einer hülsenförmigen Ausbildung des Abschnitts 17 des Eindrückteils 16, an dessen vorderem Ende die Angriffselemente 18 angeordnet sind, wäre es beispielsweise auch denkbar und möglich, diesen Abschnitt in Form von zwei oder mehreren Bolzen auszubilden, zwischen denen die Befestigungsanker 3 liegen.

Bei dem in den Figuren dargestellten Ausführungsbeispiel ist das Bedienstück 2 mehrfach verwendbar und das als Magazin dienende Endstück 1 kann nach Aufbrauch der darin enthaltenen Befestigungsanker 3 gegen ein neues Endstück 1 ausgetauscht werden, welches mit Befestigungsankern 3 befüllt ist (dies ist der Auslieferungszustand der Endstücke 1). Stattdessen könnte die Vorrichtung auch als Einweginstrument ausgebildet sein. Das Bedienstück 2 und Endstück 1 könnten in diesem Falle einstückig ausgebildet sein, wobei das Betätigungsteil 10 mit dem Nadelhalter 12 einstückig ausgebildet sein könnte. Im Auslieferungszustand ist das Einweginstrument wiederum mit Befestigungsankern 3 vollständig befüllt.

### Legende zu den Hinweisziffern:

- 1: Endstück
- 2: Bedienstück
- 3: Befestigungsanker
- 4: Schaft
- 5: vorspringender Teil
- 6: Vorsprung
- 7: Vorsprung
- 8: Durchgangskanal
- 9: Öffnung
- 10: Betätigungsteil
- 11: Vorschubrichtung
- 12: Nadelhalter
- 13: Nadel
- 14: Feder
- 15: Stützscheibe
- 16: Eindrückteil

- 17: Abschnitt
- 18: Angriffselement
- 19: Rückseite
- 20: Haltezunge
- 21: Außenhülse
- 22: Führungszapfen
- 23: Führungsschlitz
- 24: Haltezunge
- 25: Sperrelement
- 26: Übertragungsschaft
- 27: Handhabe
- 28: Krallteil
- 29: Außenteil
- 30: Federlappen
- 31: Öffnung

## Patentansprüche

1. Vorrichtung zum Setzen von Befestigungsankern in das menschliche oder tierische Gewebe, insbesondere zur Befestigung von Kunststoffnetzen, mit einem Betätigungsteil (10), das für einen Setzvorgang von einer Ausgangsstellung zu einer Vorschubstellung in eine Vorschubrichtung (11) vorschiebbar und entgegen der Vorschubrichtung (11) zu seiner Ausgangsstellung rückstellbar ist, einer vom Betätigungsteil (10) bei dessen Vorschub von der Ausgangsstellung zur Vorschubstellung vorgeschobenen und bei dessen Rückstellung von der Vorschubstellung zur Ausgangsstellung zurückgezogenen Nadel (13), die am bezogen auf die Vorschubrichtung (11) vorderen Ende eine Nadelspitze besitzt, mehreren Befestigungsankern, die jeweils einen in das Gewebe einzudrückenden Schaft (4), mindestens einen gegenüber dem Schaft (4) vorspringenden Teil (5) mit einer dem vorderen Ende der Nadel (13) abgewandten Rückseite und einen den Befestigungsanker (3) durchsetzenden Durchgangskanal (8) aufweisen und die auf der ihre Durchgangskanäle (8) durchsetzenden Nadel (13) angeordnet sind, einem beim Vorschub des Betätigungsteils (10) von dessen Ausgangsstellung zur Vorschubstellung vorgeschobenen Eindrückteil (16) zum Eindrücken des jeweils bezogen auf die Vorschubrichtung vordersten, in einer Bereitschaftsposition im Bereich der Nadelspitze sich befindenden Befestigungsankers (3) ins Gewebe und einem Nachführmechanismus zum in Richtung zur Nadelspitze gerichteten Verschieben der hinter dem ins Gewebe einzudrückenden Befestigungsanker sich befindenden Befestigungsanker (3) beim Setzvorgang, **dadurch gekennzeichnet, dass** das Eindrückteil (16) einen Abschnitt (17) aufweist, der sich im vollständig mit Befestigungsankern (3) befüllten Zustand der Vorrichtung über mehrere der Befestigungsanker (3) in Richtung zum vorderen Ende der Nadel (13) erstreckt und an dessen vorderem Ende Angriffselemente (18) zum Angriff an der Rückseite mindestens eines vorspringenden Teils (5) eines Befestigungsankers (3) angeordnet sind, und dass die Angriffselemente (18) des Eindrückteils (16) bei der Rückstellung des Betätigungsteils (10) von seiner Vorschubstellung zu seiner Ausgangsstellung über oder um mindestens einen vorspringenden Teil (5) des nächsthinteren Befestigungsankers (3) überführbar und zur Anlage an der Rückseite mindestens eines vorspringenden Teils (5) dieses nächsthinteren Befestigungsankers (3) bringbar sind.

2. Vorrichtung nach Anspruch 1, **dadurch gekennzeichnet, dass** der Abschnitt (17) des Eindrückteils (16), an dessen vorderem Ende die Angriffselemente (18) angeordnet sind, hülsenförmig ausgebildet ist.

3. Vorrichtung nach Anspruch 1 oder Anspruch 2, **dadurch gekennzeichnet, dass** das Eindrückteil (16) und die Nadel (13) in und entgegen der Vorschubrichtung (11) unverschiebbar miteinander verbunden sind.

4. Vorrichtung nach einem der Ansprüche 1 bis 3, **dadurch gekennzeichnet, dass** die Angriffselemente (18) als Zungen ausgebildet sind, die zur Überführung über oder um den mindestens einen vorspringenden Teil (5) des nächsthinteren Befestigungsankers (3) federelastisch auslenkbar sind.

5. Vorrichtung nach einem der Ansprüche 1 bis 4, **dadurch gekennzeichnet, dass** die Angriffselemente (18) des Eindrückteils (16) am jeweils vordersten auf der Nadel (13) angeordneten Befestigungsanker (3) angreifen.

6. Vorrichtung nach einem der Ansprüche 1 bis 5, **dadurch gekennzeichnet, dass** das Eindrückteil (16) verschiebbar in einer Außenhülse (21) angeordnet ist.

7. Vorrichtung nach Anspruch 6, **dadurch gekennzeichnet, dass** die Außenhülse (21) zumindest in der Ausgangsstellung des Betätigungsteils (10) alle auf der Nadel (13) angeordneten Befestigungsanker (3) umgibt.

8. Vorrichtung nach Anspruch 6 oder Anspruch 7, **dadurch gekennzeichnet, dass** die Außenhülse (21) mindestens eine federelastische Haltezunge (24) aufweist, die in der Ausgangsstellung des Betätigungsteils (10) mit mindestens einem vorspringenden Teil (5) des vordersten Befestigungsankers (3) zum Zurückhalten des vordersten Befestigungsankers (3) gegen eine Verschiebung in die Vorschubrichtung (11) zusammenwirkt.

9. Vorrichtung nach einem der Ansprüche 1 bis 8, **dadurch gekennzeichnet, dass** der Nachführmechanismus eine am bezogen auf die Vorschubrichtung (11) letzten Befestigungsanker(3) angreifende und diesen in die Vorschubrichtung (11) beaufschlagende Feder (14) aufweist.

10. Vorrichtung nach Anspruch 9, **dadurch gekennzeichnet, dass** die Feder (14) mindestens eine in einem mittleren Bereich der Feder (14) angeordnete Stützscheibe (15) besitzt, welche von der Nadel (13) durch eine Bohrung durchsetzt wird.

11. Vorrichtung nach Anspruch 9 oder Anspruch 10, **dadurch gekennzeichnet, dass** das Eindrückteil (16) mindestens eine federelastische Haltezunge (20) aufweist, welche in der Ausgangsstellung des Betätigungsteils (10) mit mindestens einem vorspringenden Teil (5) desjenigen Befestigungsankers (3), der dem Befestigungsanker (3) nachfolgt, an dem die Angriffselemente (18) des Eindrückteils (16) angreifen, zum Zurückhalten dieses Befestigungsankers (3) gegen eine von der Feder (14) in die Vorschubrichtung (11) erfolgende Verschiebung zusammenwirken.

12. Vorrichtung nach einem der Ansprüche 6 bis 11, **dadurch gekennzeichnet, dass** die Außenhülse (21) mindestens ein Sperrelement (25) aufweist, von dem bei der Rückstellung des Betätigungsteils (10) von seiner Vorschubstellung zu seiner Ausgangsstellung eine Verschiebung des nächsthinteren Befestigungsankers (3) entgegen der Vorschubrichtung (11) blockiert wird, wobei eine Überführung der Angriffselemente (18) des Eindrückteils (16) über oder um mindestens einen vorspringenden Teil (5) des nächsthinteren Befestigungsankers (3) erfolgt.

13. Vorrichtung nach Anspruch 12, **dadurch gekennzeichnet, dass** durch die Blockierung des Befestigungsankers (3) bei der Rückstellung des Betätigungsteils (10) auch eine Überführung der mindestens einen federelastischen Haltezunge (20) des Eindrückteils (16) unter federelastischer Auslenkung derselben über oder um den mindestens einen vorspringenden Teil des Befestigungsankers (3) erfolgt.

## Claims

1. A device for the placing of securing anchors into human or animal tissue, in particular for the securing of plastics meshes, having an actuating part (10) which for a placing procedure can be advanced from an initial position to a feed position in a feed direction (11) and can be restored contrary to the feed direction (11) to its initial position, a needle (13) which is advanced by the actuating part (10) upon advancing of the latter from the initial position to the feed position and is retracted upon restoration of the actuating part (10) from the feed position to the initial position and which has a needle point at the front end related to the feed direction (11), a plurality of securing anchors which each have a shaft (4), to be pushed into the tissue, at least one part (5), projecting with respect to the shaft (4) and having a rear side remote from the front end of the needle (13), and a through channel (8), passing through the securing anchor (3), and which are arranged on the needle (13) passing through their through channels (8), a pushing part (16), advanced upon advancing of the actuating part (10) from its initial position to the feed position, to push the securing anchor (3), foremost in each case related to the feed direction and located in a ready position in the region of the needle point, into the tissue, and a movement-following mechanism for the displacement, directed in the direction of the needle point, during the placement procedure of the securing anchors (3) located behind the securing anchor to be pushed into the tissue, **characterised in that** the pushing part (16) has a portion (17) which, when the device is in the state in which it is completely filled with securing anchors (3), extends over a plurality of the securing anchors (3), in the direction of the front end of the needle (13), and at the front end of which there are arranged contact elements (18) to contact the rear side of at least one projecting part (5) of a securing anchor (3), and **in that** upon restoration of the actuating part (10) from its feed position to its initial position, the contact elements (18) of the pushing part (16) can be conveyed over or around at least one projecting part (5) of the next-behind securing anchor (3) and can be brought into contact with the rear side of at least one projecting part (5) of this next-behind securing anchor (3).

2. A device according to claim 1, **characterised in that** the portion (17), at the front end of which the contact elements (18) are arranged, of the pushing part (16) is sleeve-shaped.

3. A device according to claim 1 or claim 2, **characterised in that** the pushing part (16) and the needle (13) are nondisplaceably connected to one another in and contrary to the feed direction (11).

4. A device according to any one of claims 1 to 3, **characterised in that** the contact elements (18) are in the form of tongues which are resiliently deflectable for conveying over or around the at least one projecting part (5) of the next-behind securing anchor (3).

5. A device according to any one of claims 1 to 4, **characterised in that** the contact elements (18) of the pushing part (16) contact the securing anchor (3) located foremost on the needle (13) in each case.

6. A device according to any one of claims 1 to 5, **characterised in that** the pushing part (16) is displaceably arranged in an outer sleeve (21).

7. A device according to claim 6, **characterised in that** at least in the initial position of the actuating part (10), the outer sleeve (21) surrounds all securing anchors (3) arranged on the needle (13).

8. A device according to claim 6 or claim 7, **characterised in that** the outer sleeve (21) has at last one resilient holding tongue (24) which, in the initial position of the actuating part (10), cooperates with at least one projecting part (5) of the foremost securing anchor (3), in order to restrain the foremost securing anchor (3) against displacement in the feed direction (11).

9. A device according to any one of claims 1 to 8, **characterised in that** the movement-following mechanism has a spring (14) which contacts the last securing anchor (3) related to the advance direction (11) and which acts thereupon in the feed direction (11).

10. A device according to claim 9, **characterised in that** the spring (14) has at least one supporting disc (15) arranged in a middle region of the spring (14), the needle (13) passing through the supporting disc (15) through a bore.

11. A device according to claim 9 or claim 10, **characterised in that** the pushing part (16) has at least one resilient holding tongue (20) which, in the initial position of the actuating part (10), cooperates with at least one projecting part (5) of that securing anchor (3) which comes after the securing anchor (3) on which the contact elements (18) of the pushing part (16) contact, in order to restrain this securing anchor (3) against displacement taking place in the feed direction (11) by the spring (14).

12. A device according to any one of claims 6 to 11, **characterised in that** the outer sleeve (21) has at least one locking elements (25) by which, upon restoration of the actuating part (10) from its feed position to its initial position, displacement of the next-behind securing anchor (3) contrary to the feed direction (11) is blocked, wherein conveying of the contact elements (18) of the pushing part (16) over or around at least one projecting part (5) of the next-behind securing anchor (3) takes place.

13. A device according to claim 12, **characterised in that** through the blocking of the securing anchor (3), upon restoration of the actuating part (10) there also takes place a conveying of the at least one resilient holding tongue (20) of the pushing part (16), accompanied by resilient deflection thereof, over or around the at least one projecting part of the securing anchor (3).

## Revendications

1. Dispositif de pose d'ancrages de fixation dans le tissu humain ou animal, en particulier pour la fixation de filets en matière synthétique, comportant une partie d'actionnement (10) qui peut être avancée pour un processus de pose, d'une position de départ à une position d'avancée dans un sens d'avancée (11) et peut être reculée dans le sens opposé au sens d'avancée (11) à sa position de départ, une aiguille (13) avancée par la partie d'actionnement (10) lors de son avancée de la position de départ à la position d'avancée et retirée lors de son recul de la position d'avancée à la position de départ, qui possède une pointe d'aiguille à l'extrémité avant par rapport au sens d'avancée (11), plusieurs ancrages de fixation qui présentent respectivement une tige (4) à enfoncer dans le tissu, au moins une partie (5) proéminente en face de la tige (4) comportant une face arrière détournée de l'extrémité avant de l'aiguille (13) et un canal de passage (8) traversant l'ancrage de fixation (3), et qui sont disposés sur l'aiguille (13) traversant leurs canaux de passage (8), une partie d'enfoncement (16) avancée lors de l'avancée de la partie d'actionnement (10) de sa position de départ à la position d'avancée pour enfoncer l'ancrage de fixation (3) se trouvant respectivement par rapport au sens d'avancée, dans une position de mise à disposition dans la région de la pointe de l'aiguille dans le tissu, et un mécanisme d'asservissement pour déplacer de façon orientée dans la direction de la pointe de l'aiguille, l'ancrage de fixation (3) se trouvant derrière l'ancrage de fixation s'enfonçant dans le tissu lors du processus de pose, **caractérisé en ce que** la partie d'enfoncement (16) présente un segment (17) qui s'étend à l'état complètement rempli par les ancrages de fixation (3) du dispositif d'avancée, sur plusieurs des ancrages de fixation (3) dans la direction de l'extrémité avant de l'aiguille (13) et sur son extrémité avant sont disposés des éléments d'intervention (18) pour intervenir sur la face arrière d'au moins une partie proéminente (5) d'un ancrage de fixation (3), et **en ce que** les éléments d'intervention (18) de la partie d'enfoncement (16) peuvent passer lors du recul de la partie d'actionnement (10) de sa position d'avancée à sa position de départ sur ou autour d'au moins une partie proéminente (5) de l'ancrage de fixation (3) arrière suivant et peuvent être placés sur la face arrière d'au moins une partie proéminente (5) de cet ancrage de fixation arrière suivant (3).

2. Dispositif selon la revendication 1, **caractérisé en ce que** le segment (17) de la partie d'enfoncement (16) à l'extrémité avant duquel les éléments d'intervention (18) sont disposés, sont constitués en forme de gaine.

3. Dispositif selon la revendication 1 ou la revendication 2, **caractérisé en ce que** la partie d'enfoncement (16) et l'aiguille (13) sont reliées l'une à l'autre dans et à l'opposé du sens d'avancée (11) de façon non déplaçable.

4. Dispositif selon l'une des revendications 1 à 3, **caractérisé en ce que** les éléments d'intervention (18) sont constitués comme des languettes déformables élastiquement pour passer sur ou autour d'au moins une partie proéminente (5) de l'ancrage de fixation (3) arrière suivant.

5. Dispositif selon l'une des revendications 1 à 4, **caractérisé en ce que** les éléments d'intervention (18) de la partie d'enfoncement (16) interviennent sur l'ancrage de fixation (3) disposé respectivement à l'avant sur l'aiguille (13).

6. Dispositif selon l'une des revendications 1 à 5, **caractérisé en ce que** la partie d'enfoncement (16) est disposée de façon à pouvoir être poussée dans une gaine extérieure (21).

7. Dispositif selon la revendication 6, **caractérisé en ce que** la gaine extérieure (21) entoure au moins dans la position de départ de la partie d'actionnement (10) tous les ancrages de fixation disposés sur l'aiguille (13).

8. Dispositif selon la revendication 6 ou la revendication 7, **caractérisé en ce que** la gaine extérieure (21) présente au moins une languette de retenue élastique (24) qui agit en position de départ de la partie d'actionnement (10) avec au moins une partie proéminente (5) de l'ancrage de fixation avant (3) pour retenir l'ancrage de fixation avant (3) contre un déplacement dans le sens d'avancée (11).

9. Dispositif selon l'une des revendications 1 à 8, **caractérisé en ce que** le mécanisme d'asservissement présente un ressort (14) intervenant sur le dernier ancrage de fixation (3) par rapport au sens d'avancée (11) et actionnant celui-ci dans le sens d'avancée.

10. Dispositif selon la revendication 9, **caractérisé en ce que** le ressort (14) possède au moins une rondelle d'appui (15) disposé dans une zone médiane du ressort (14) qui est traversé par l'aiguille (13) dans un alésage.

11. Dispositif selon la revendication 9 ou la revendication 10, **caractérisé en ce que** la partie d'enfoncement (16) présente au moins une languette de retenue (20) élastique qui coopère en position de départ de la partie d'actionnement (10) comportant au moins une partie proéminente (5) de l'ancrage de fixation (3) qui suit l'ancrage de fixation (3) sur lequel les éléments d'intervention (18) de la partie d'enfoncement (16) interviennent, pour retenir cet ancrage de fixation (3) contre un déplacement s'effectuant par le ressort (14) dans le sens d'avancée (11).

12. Dispositif selon l'une des revendications 6 à 11, **caractérisé en ce que** la gaine extérieure (21) présente au moins un élément de blocage (25) par lequel lors du retrait de la partie d'actionnement (10) de sa position d'avancée à sa position de départ, un déplacement de l'ancrage de fixation arrière suivant (3) est bloqué contre l'avancée (11), un passage des éléments d'intervention (18) de la partie d'enfoncement (16) sur ou autour d'au moins une partie proéminente (5) de l'ancrage de fixation arrière suivant s'effectuant.

13. Dispositif selon la revendication 12, caractérisé en ce le blocage de l'ancrage de fixation (3) lors du recul de la partie d'actionnement (10) permet également un passage d'au moins une languette de retenue élastique (20) de la partie d'enfoncement (16) par déformation élastique de celle-ci sur ou autour au moins d'une partie proéminente de l'ancrage de fixation (3).
